# EUROPEAN PATENT APPLICATION

(11) **EP 4 595 889 A1**
(43) Date of publication of application: **06.08.2025**
(21) Application number: 25150941.0
(22) Date of filing: 09.01.2025
(51) Int. Cl.: A61B 6/03, A61B 6/04

(54) **WIDE ANGLE, PINCHLESS PATIENT POSITIONING CRADLE FOR A MEDICAL IMAGING SYSTEM**

(30) Priority: 02.02.2024 US 202418431273
(71) Applicant: GE Precision Healthcare LLC, Waukesha, WI 53188 (US)
(72) Inventor: NAYAK K, Vishwanath, 560066 Bangalore (IN); RAO, Ramachandra Gururaja, 560066 Bangalore (IN); DANGASHIYA, Dhaval Pravinbhai, 560066 Bangalore (IN)
(74) Representative: AWA Sweden AB

(57) **Abstract**

A patient table (46) for a medical imaging system includes a base (52). The patient table (46) also includes a cradle (50) configured to support a subject to be imaged and to move bi-directionally relative to the base (52). The patient table (46) further includes a fixed structure (78) coupled to both the base (52) and the cradle (50). The cradle (50) is configured to move relative to the fixed structure (78) without a portion of the subject being pinched between the cradle (50) and the fixed structure (78).

## Description

### BACKGROUND

The subject matter disclosed herein relates to imaging systems and, more particularly, to a wide angle, pinchless patient positioning cradle for a medical imaging system.

Non-invasive imaging technologies allow images of the internal structures or features of a patient to be obtained without performing an invasive procedure on the patient. In particular, such non-invasive imaging technologies rely on various physical principles, such as the differential transmission of X-rays through the target volume or the reflection of acoustic waves, to acquire data and to construct images or otherwise represent the observed internal features of the patient.

For example, in computed tomography (CT) and other X-ray based imaging technologies, X-ray radiation spans and is transmitted through a subject of interest, such as a human patient, and a portion of the X-ray radiation impacts an X-ray detector where image data is collected. In digital X-ray systems a photodetector produces signals representative of the amount or intensity of X-ray radiation impacting discrete pixel regions of X-ray detector elements or sensors. The signals may then be processed to generate an image that may be displayed for review. In CT imaging systems, an X-ray detector array, including a series of detector elements or sensors, produces similar signals through various positions as a gantry housing an X-ray source and the X-ray detector array is rotated around a patient.

A patient is moved into and out of a bore or opening extending through the center of a gantry of the CT scanner or other medical imaging system via a patient cradle that is part of a patient table. In particular, a typical patient table typically includes a long stationary beam structure (e.g., extruded beam structure) with side support structures that flank and support a patient supporting cradle. The long stationary beam structure has linear motion guide rails mounted thereon that guide the patient supporting cradle during its movement into and out of the gantry. Typically, this configuration may cause a patient to pinch their body parts due to a gap between the moving component (e.g., patient supporting cradle) and the fixed structure (e.g., stationary beam structure). In addition, this gap between the side support structures and the patient supporting cradle is susceptible to collecting and entrapping dust, foreign particles, patient fluids, and other material which is difficult to disinfect and clean due to lack of accessibility. Further, the side support structures are typically made of metal that provides a cold shock to a patient getting on the table when they contact the metal. Even further, a width of the cradle is limited due to the need of the side support structures for loading and unloading of the patient onto the patient table as the side support structures are not configured to take a single patient point load of a patient getting onto or loading on the table. Still further, the patient supporting cradle causes patient discomfort (e.g., due to patient overhang) during motion of the patient supporting cradle. Further yet, the patient supporting cradle (as well as a lower resting surface area for the patient supporting cradle to accommodate heavier patients) may cause kinesiophobia and/or claustrophobia due to the patient being able to see movement of the patient supporting cradle relative to the side support structures (which also may lead to entrapment of a patient's fingers, hand or other body parts in the gap due to a patient panicking and grabbing the cradle/support structure). Further, the typical patient supporting cradle has a foam core (e.g., due to the greater depth of the cradle) which increases the depth and thickness of the cradle which causes a higher X-ray dose for the patient due to the higher attenuation by the cradle foam core cross-section. Even further, it is difficult to conduct radiation therapy procedures with a typical patient table due to the structural limitations imposed by the presence of the side support structures.

### SUMMARY

Certain embodiments commensurate in scope with the originally claimed subject matter are summarized below. These embodiments are not intended to limit the scope of the claimed subject matter, but rather these embodiments are intended only to provide a brief summary of possible forms of the subject matter. Indeed, the subject matter may encompass a variety of forms that may be similar to or different from the embodiments set forth below.

In one embodiment, a patient table for a medical imaging system is provided. The patient table includes a base. The patient table also includes a cradle configured to support a subject to be imaged and to move bi-directionally relative to the base. The patient table further includes a fixed structure coupled to both the base and the cradle. The cradle is configured to move relative to the fixed structure without a portion of the subject being pinched between the cradle and the fixed structure.

In another embodiment, a computed tomography (CT) imaging system is provided. The CT imaging system includes a gantry having a bore and coupled to imaging components configured to acquire imaging data of a subject. The CT imaging system also includes a patient table. The patient table includes a base. The patient table further includes a fixed structure coupled to both the base and the cradle. The patient table also includes a cradle configured to support a subject to be imaged and to move bi-directionally relative to the base. The cradle has a first width in a first direction perpendicular to a longitudinal axis of the cradle. The first width is greater than a second width of the fixed structure in the first direction.

In a further embodiment, a patient table for an imaging system is provided. The patient table includes a base. The patient table also includes a cradle configured to support a subject to be imaged and to move bi-directionally relative to the base. The patient table further includes a fixed structure coupled to both the base and the cradle. The cradle includes a top surface and a bottom surface. The cradle also includes a first section and a second section along a longitudinal axis of the cradle. The first section is configured to be extended beyond the fixed structure while the second section is configured to remain located above the fixed structure when the first section is extended beyond the fixed structure. The first section has a first thickness in a first direction between the top surface and the bottom surface along the longitudinal axis and the second section has a second thickness in the first direction along the longitudinal axis. The second thickness is greater than the first thickness.

### BRIEF DESCRIPTION OF THE DRAWINGS

These and other features, aspects, and advantages of the disclosed subject matter will become better understood when the following detailed description is read with reference to the accompanying drawings in which like characters represent like parts throughout the drawings, wherein:
FIG. 1 is a combined pictorial view of a patient beginning to load on a cradle of a prior art patient table and the patient loaded on the cradle of the prior art patient table;
FIG. 2 is a perspective view of a patient table that may be used with a medical imaging system in accordance with aspects of the present disclosure;
FIG. 3 is a top view of the patient table depicted in FIG. 2, in accordance with aspects of the present disclosure;
FIG. 4 is an end view of a portion of the patient table depicted in in FIG. 2, in accordance with aspects of the present disclosure;
FIG. 5 is an end view of a cradle of the patient table depicted in FIG. 2, in accordance with aspects of the present disclosure;
FIG. 6 is a top view of a patient disposed on the cradle of the patient table depicted in FIG. 2, in accordance with aspects of the present disclosure;
FIG. 7 is a perspective view of a cradle of the patient table depicted in FIG. 2, in accordance with aspects of the present disclosure;
FIG. 8 is an end view of a cradle of the patient table depicted in FIG. 2 (e.g., having a first width), in accordance with aspects of the present disclosure;
FIG. 9 is an end view of a cradle of the patient table depicted in FIG. 2 (e.g., having a second width), in accordance with aspects of the present disclosure;
FIG. 10 is a top view of a cradle of the patient table depicted in FIG. 2 (e.g., having respective dimensions), in accordance with aspects of the present disclosure;
FIG. 11 is an end view of the cradle in FIG. 10 and table with associated values, in accordance with aspects of the present disclosure;
FIG. 12 depicts a patient beginning to load on a cradle of the patient table in FIG. 2, in accordance with aspects of the present disclosure;
FIG. 13 depicts a patient loaded on a cradle of the patient table in FIG. 2, in accordance with aspects of the present disclosure;
FIG. 14 depicts analysis of a load case (e.g., patient climb load case) for the cradle in FIG. 10, in accordance with aspects of the present disclosure;
FIG. 15 depicts the attenuation at various points during CT imaging of a patient utilizing a typical cradle;
FIG. 16 depicts a cradle of the patient table in FIG. 2 with respect to path of X-rays passing through a patient during a scan of a patient, in accordance with aspects of the present disclosure;
FIG. 17 depicts the attenuation at various points during CT imaging of a patient utilizing a cradle of the patient table in FIG. 2, in accordance with aspects of the present disclosure;
FIG. 18 depicts a table of results for analyzing X-ray attenuation for both a typical cradle and a cradle of the patient table depicted in FIG. 2, in accordance with aspects of the present disclosure; and
FIG. 19 depicts CT images of both a typical prior art cradle depicted in FIG. 1 and a cradle of the present disclosure depicted in FIG. 2.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

While aspects of the following discussion are provided in the context of medical imaging, it should be appreciated that the disclosed techniques are not limited to such medical contexts. Indeed, the provision of examples and explanations in such a medical context is only to facilitate explanation by providing instances of real-world implementations and applications. However, the disclosed techniques may also be utilized in other contexts, such as image reconstruction for non-destructive inspection of manufactured parts or goods (i.e., quality control or quality review applications), and/or the non-invasive inspection of packages, boxes, luggage, and so forth (i.e., security or screening applications). In general, the disclosed techniques may be useful in any imaging or screening context or image processing or photography field where a set or type of acquired data undergoes a reconstruction process to generate an image or volume.

The present disclosure provides embodiments for a patient table of medical imaging systems (e.g., a computed tomography (CT) imaging system, magnetic resonance imaging (MRI) system, positron emission tomography (PET) imaging system, single-photon emission computed tomography (SPECT) imaging system, nuclear medicine imaging system, X-ray imaging system, or any combinations thereof etc.) that includes a wide angle, pinchless patient positioning and patient supporting cradle. The patient table includes a base. In certain embodiments, the base is fixed. In certain embodiments, the base is configured to move in a vertical and/or horizontal direction relative to a floor. The patient table also includes a cradle configured to support a subject (e.g., patient) to be imaged and to move bi-directionally relative to the base. The patient table further includes a fixed structure coupled to both the base and the cradle. The cradle is configured to move relative to the fixed structure without a portion of the subject being pinched between the cradle and the fixed structure.

In certain embodiments, the cradle has a first width in a first direction perpendicular to a longitudinal axis (or longitudinal length) of the cradle, and the first width is greater than a second width of the first structure in the first direction. In certain embodiments, the cradle is located above the fixed structure along the first width. In certain embodiments, the first width is at least 52 centimeters (cm). In certain embodiments, a cross-section of the cradle along the first width includes a plurality of sections with at least two sections sloped relative to a central section of the plurality of sections.

In certain embodiments, cradle varies in thickness along the longitudinal axis both in the first direction (e.g., lateral direction or width) and in a second direction (e.g., between a top surface and a bottom surface of the cradle) perpendicular to the first direction. In certain embodiments, the cradle includes a first section and a second section along the longitudinal axis. The first section is configured to be extended beyond the fixed structure (e.g., into a bore of a gantry) while the second section is configured to remain located above the fixed structure (e.g., outside the bore of the gantry) when the first section is extended beyond the fixed structure. The first section has a first thickness in the second direction along the longitudinal axis and the second section has a second thickness in the second direction along the longitudinal axis. The second thickness is greater than the first thickness.

In certain embodiments, the cradle is configured to support a single subject (e.g., patient) load to enable the subject both to load and to unload onto the cradle solely utilizing the cradle. In certain embodiments, the cradle is configured to provide a flat surface for engagement with a radiation therapy table top. In certain embodiments, the cradle lacks a foam core. In certain embodiments, the cradle has a lower X-ray attenuation than another cradle having the foam core. In certain embodiments, the cradle is made of carbon fiber reinforced plastic. In certain embodiments, the cradle is manufactured utilizing a carbon fiber reinforced lay-up process.

The disclosed embodiments provide a patient table having a cradle and lacking side support structures to provide a pinchless configuration where no portion of a subject will be pinched between the cradle and the fixed structure during movement of the cradle relative to the fixed structure. The cradle itself serves as the support structure for patient positioning on the cradle without the need of the side support structures. The disclosed configuration of the table eliminates any need for additional external support or structure to hold the cradle and position the cradle. The elimination of the side support structures and the wider cradle keeps the patient from experiencing kinesiophobia and claustrophobia. The disclosed embodiments further provide a patient table that does not provide a cold shock to the patient. Thus, the disclosed embodiments provide a better patient experience. The disclosed embodiments also provide a patient able with improved cleanability and disinfection. This will reduce associated repair issues and, thus, less downtime for the medical imaging system. The disclosed embodiments even further provide a wider cradle that a flat surface for engagement with a radiation therapy table top. The disclosed embodiments still further provide a thin, strong, and cost efficient part (i.e., the cradle) made of carbon fiber reinforced plastic. A simple carbon fiber reinforced plastic lay-up process for manufacturing the cradle improves production. The disclosed embodiments event further include subjecting a patient to a lower X-ray dose due to the cradle having a lower X-ray attenuation. This improves the life of the X-ray tube and improves the overall system throughput.

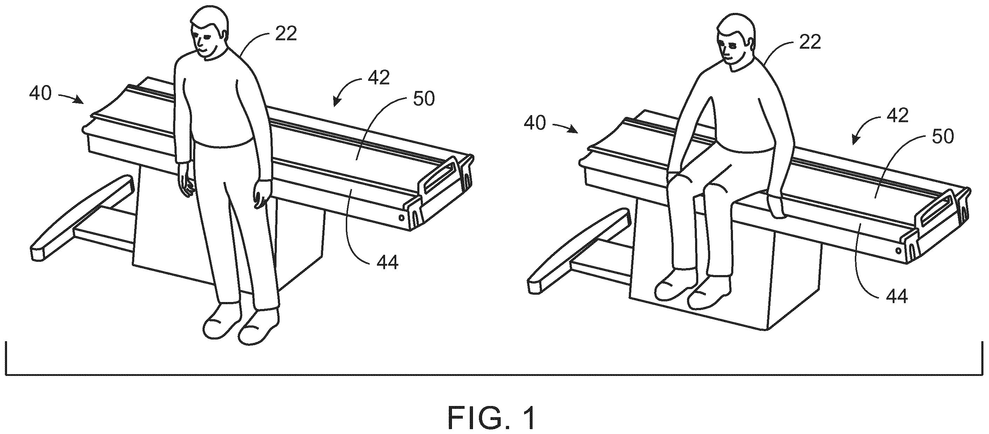

FIG. 1 is a combined pictorial view of a patient 22 beginning to load on a cradle 50 of a prior art patient table 40 (e.g., for a CT imaging system) and the patient 22 loaded on the cradle 50 of the prior art patient table 40. The prior art patient table 40 typically includes a long stationary beam structure 42 (e.g., extruded beam structure) with side support structures 44 that flank and support the patient supporting cradle 50. The long stationary beam structure 42 has linear motion guide rails mounted thereon that guide the patient supporting cradle 50 during its movement into and out of the gantry. Typically, this configuration may cause the patient 22 to pinch their body parts due to a gap between the moving component (e.g., patient supporting cradle 50) and the fixed structure (e.g., stationary beam structure 42). In addition, this gap between the side support structures 44 and the patient supporting cradle 50 is susceptible to collecting and entrapping dust, foreign particles, patient fluids, and other material which is difficult to disinfect and clean due to lack of accessibility. Further, the side support structures 44 are typically made of metal that provides a cold shock to the patient 22 getting on the table 40 when they contact the metal. Even further, a width of the cradle 50 is limited due to the need of the side support structures 44 for loading and unloading of the patient 22 onto the patient table 40 as the side support structures 44 are not configured to take a single patient point load of the patient 22 getting onto or loading on the table 40. Still further, the patient supporting cradle 50 causes patient discomfort (e.g., due to patient overhang) during motion of the patient supporting cradle 50. Further yet, the patient supporting cradle 50 (as well as a lower resting surface area for the patient supporting cradle 50 to accommodate heavier patients) may cause kinesiophobia and/or claustrophobia due to the patient 22 being able to see movement of the patient supporting cradle 50 relative to the side support structures 44 (which also may lead to entrapment of a patient's fingers, hand or other body parts in the gap due to the patient panicking 22 and grabbing the cradle/support structure). Further, the typical patient supporting cradle 50 has a foam core (e.g., due to the greater depth of the cradle 50) which increases the depth and thickness of the cradle 50 which causes a higher X-ray dose for the patient 22 due to the higher attenuation by the cradle foam core cross-section. Even further, it is difficult to conduct radiation therapy procedures with the prior art patient table 40 due to the structural limitations imposed by the presence of the side support structures 44.

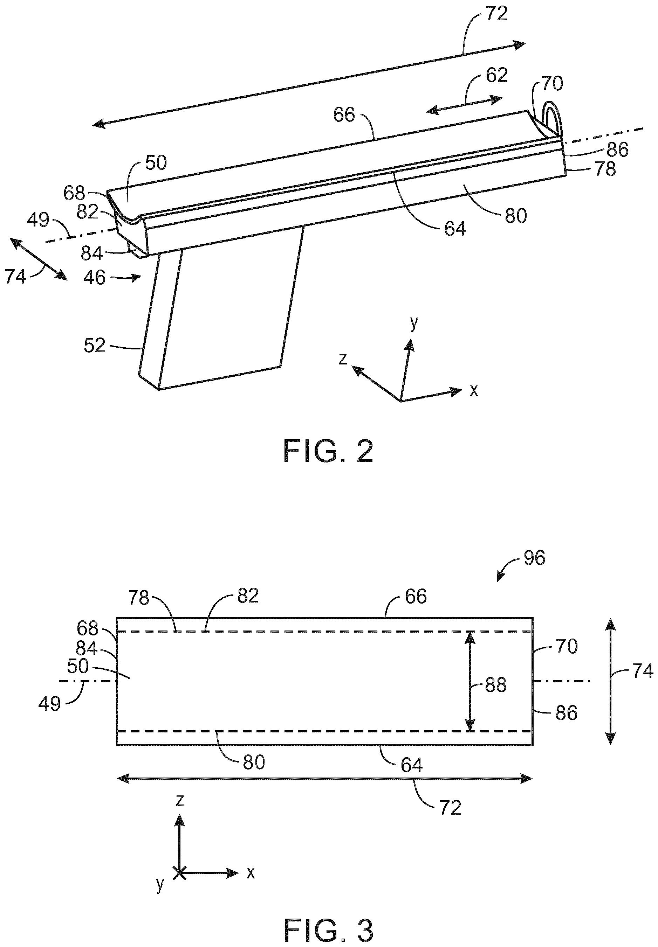

Although a patient table 46 (see FIGS. 2 and 3) in the present disclosure may be discussed in the context of a CT imaging system, the patient table 46 may be utilized with other types of medical imaging systems (e.g., magnetic resonance imaging (MRI), positron emission tomography (PET) imaging system, single-photon emission computed tomography (SPECT) imaging system, nuclear medicine imaging system, X-ray imaging system, etc.). That is, the depicted example of the CT imaging system 10 is merely one environment in which the described patient table 46 may be implemented. Aspects of the patient table 46 in the following figures are discussed utilizing a coordinate system having a y-direction (or y-axis), an x-direction (or x-axis), and a z-direction (or z-axis). The coordinate system may be discussed relative to a longitudinal axis 49 of the patient table 46 (and floating cradle 50). FIGS. 2 and 3 are different views of the patient table 46 of the CT imaging system 10 in FIG. 1. The patient table 46 includes a base 52. In certain embodiments, the base 52 is fixed to a single location on a floor in a scan room. In other words, the base 52 does not move in the y-direction, the x-direction, and the z-direction. In certain embodiments, the base 52 is configured to move bi-directionally along a path (e.g., extending in the x-direction or axial direction) on the floor in the scan room (e.g., via linear motion guide rails). The structure of the base 52 may vary from that depicted in FIGS. 2 and 3.

The patient table 46 also includes the patient supporting cradle 50. The cradle 50 is configured to support a subject (e.g., patient) to be imaged. The cradle 50 is configured to move bi-directionally (as indicated by arrow 62) relative to the base 52 in the x-direction and along the longitudinal axis 49. The cradle 50 includes a first side 64 and a second side 66 both extending between a first end 68 (first longitudinal end) and a second end 70 (second longitudinal end) in the x-direction. The first end 68 is configured to face the gantry of the imaging system and configured to be moved in and out of the bore of the gantry. The cradle 50, the first side 64, and the second side 66 have a length 72 (e.g., longitudinal length) in the x-direction. As discussed in greater detail below, the cradle varies in thickness along the longitudinal direction. The cradle 50, the first end 68, the second end 70 have a width 74 in the z-direction. In certain embodiments, the width 74 is at least 52 cm. In certain embodiments, the width 74 may range between 42 cm and 56 cm (or greater). In certain embodiments, the width 74 may be 52 cm. In certain embodiments, the width 74 may be 54 cm. In certain embodiments, the width 74 may be 56 cm. The width 74 of the cradle 50 is wide enough so that every portion of the patient (of patients of all sizes) is disposed on the cradle 50 so that no portion of the patient overhangs the cradle 50.

The patient table 46 further includes a fixed structure 78 coupled to a top of the base 52 (e.g., top portion 58). In FIG. 3, the fixed structure 78 is represented by a dashed line. Various roller supports (not shown) associated with the fixed structure 78 facilitate movement of the cradle relative to the fixed structure 78. The fixed structure 78 does not move relative to the top of the base 52 or the entirety of the base 52 in any direction. The entirety of the fixed structure 78 is located beneath the cradle 50 in the y-direction. The fixed structure 78 includes a first side 80 and a second side 82 both extending between a first end 84 (first longitudinal end) and a second end 86 (second longitudinal length) in the x-direction. The fixed structure 78, the first end 84, and the second end 86 have a width 88 in the z-direction.

The patient table 46 lacks side support structures for the cradle 50. The patient table 46 does not have any additional external support or structure to hold the cradle 50 and position the 50 . The width 74 of the cradle 50 is greater than the width 88 of the fixed structure 78. The cradle 70 is located above the fixed structure 78 along the width 88. Due to no side support structure flanking the cradle 50, the cradle 50 is configured to move relative to the fixed structure 78 without a portion (e.g., finger or hand) getting pinched between the cradle 50 and the fixed structure 78. In addition, the width 74 of the cradle (and the absence of any side support structure) keeps the subject from experiencing kinesiophobia and claustrophobia due to not visually observing stationary and moving table parts moving relative to each other during cradle motion.

The cradle 50 is made of carbon fiber reinforced plastic. In particular, the cradle 50 is manufactured via a carbon fiber reinforced (or any composite or X-ray transparent material) lay-up process. This manufacturing technique provides a cradle at a lower cost and improves productivity (comparted to typical cradles). When the patient loads onto the cradle they do not experience a cold shock (e.g., due to cradle 50 not being made of metal). In certain embodiments, the cradle 50 lacks a foam core. In certain embodiments, the cradle 50 has a lower X-ray attenuation than another cradle having a foam core. In certain embodiments, the cradle 50 may be thinner and slimmer than typical cradles. As discussed in greater detail below, the cradle 50 is configured to support a single subject (e.g., patient) load to enable the subject both to load and to unload onto the cradle 50 solely utilizing the cradle 50. In certain embodiments, a collapsible handle structure may be disposed adjacent to the cradle 50 to help the subject to load and to unload onto the cradle 50.

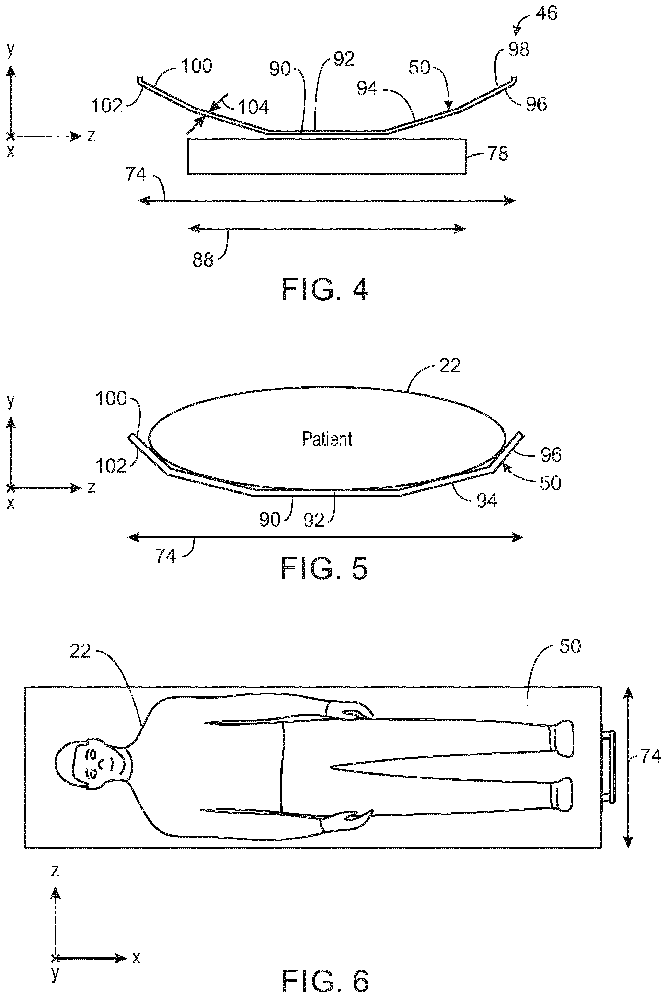

FIG. 4 is an end view of a portion of the patient table 46 depicted in FIG. 2. FIG. 5 is an end view of the cradle 50 of the patient table 46 depicted in FIG. 2. As noted above, the cradle 50 is located above the fixed structure 78 as shown in FIG. 5. Also, the width 74 of the cradle 50 is greater than the width 88 of the fixed structure 78. In certain embodiments, the width 74 is at least 42 cm. In certain embodiments, the width 74 may range between 42 cm and 56 cm (or greater). In certain embodiments, the width 74 may be 52 cm. In certain embodiments, the width 74 may be 54 cm. In certain embodiments, the width 74 may be 56 cm. The width 74 of the cradle 50 is wide enough so that every portion of the patient (of patients of all sizes) is disposed on the cradle 50 so that no portion of the patient 22 overhangs the cradle 50 as shown in FIGS. 5 and 6. In certain embodiments, the width 88 of the fixed structure 78 may be greater than the width 74 of the cradle 50. As depicted, the cradle 50 lacks a foam core. In certain embodiments, the cradle 50 may include a foam core. As depicted, the patient table 46 lacks side support structures to provide a pinchless configuration where no portion of the patient 22 will be pinched between the cradle 50 and the fixed structure 78 during movement of the cradle 50 relative to the fixed structure 78.

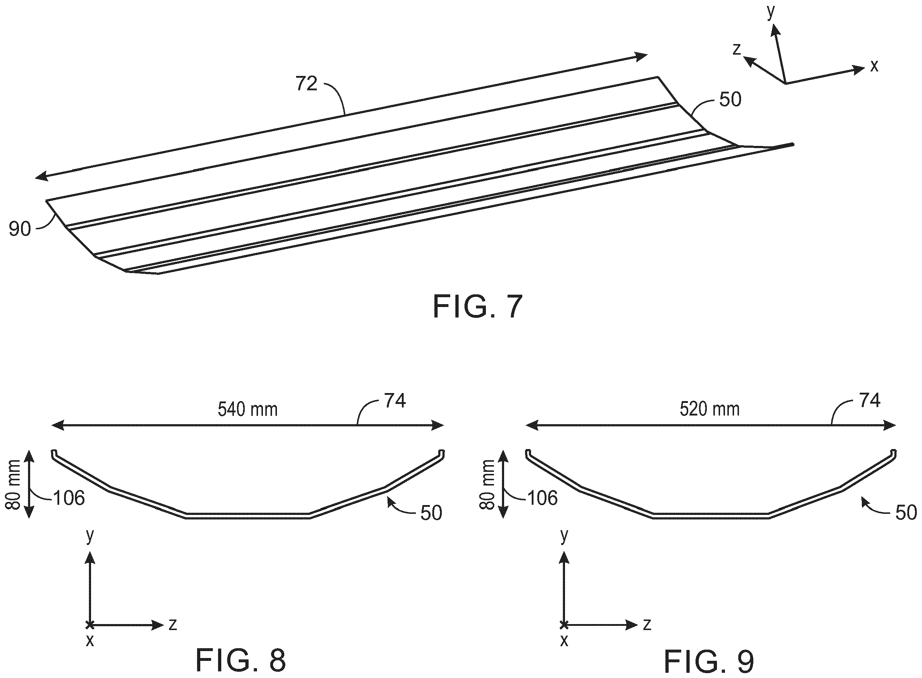

As depicted in FIGS. 4 and 5, the profile along the width 74 (or cross-section) of the cradle 50 varies. As depicted, a cross-section of the cradle 50 along the width 74 includes a plurality of sections 90. The cradle 50 along the width 74 may include 3 or more sections 90. Each section 90 is flat and extends in the x-direction. Each section 90 also extends along the longitudinal length 72 (as depicted in FIG. 7) of the cradle 50. As depicted, the cradle 50 includes at least 5 sections 90. The cradle 50 includes a central section 92, a first pair of sections 94 flank the central section 92, and a second pair of sections 96 flank both the central section 92 and the first pair of sections 94. The central section 92 is horizontal. The first pair of sections 94 are angled or sloped relative to the central section 92. The second pair of sections 96 are angle or sloped relative to both the central section 92 and the first pair of sections 94. The slope or angle of the second pair of sections 96 relative to central section 92 is greater than the slope or angle of the first pair of sections 94 relative to the central section 92. In certain embodiments, the cradle 50 along the width 74 may have a smooth curve. In certain embodiments, the cradle 50 along the width 74 may have any shape. In certain embodiments, a width of one or more of the sections 90 may vary from each other in the x-direction. In certain embodiments, the width of the plurality of sections 90 may be the same. In certain embodiments, as depicted in FIG. 4, a respective vertical section 98 (extending in the z-direction) may be disposed on the outermost portion of each section 96 of the pair of sections 96. The vertical sections 98 extend along the longitudinal length 72. The arrangement of the sections 90 makes the cradle 50 more comfortable for the patient. The flat and horizontal central section 92 provides a flat surface for engagement with a radiation therapy table top to enable the cradle 50 to be utilized easier with a radiation therapy scan. As depicted, the cradle 50 lacks a foam core. In certain embodiments, the cradle 50 may include a foam core.

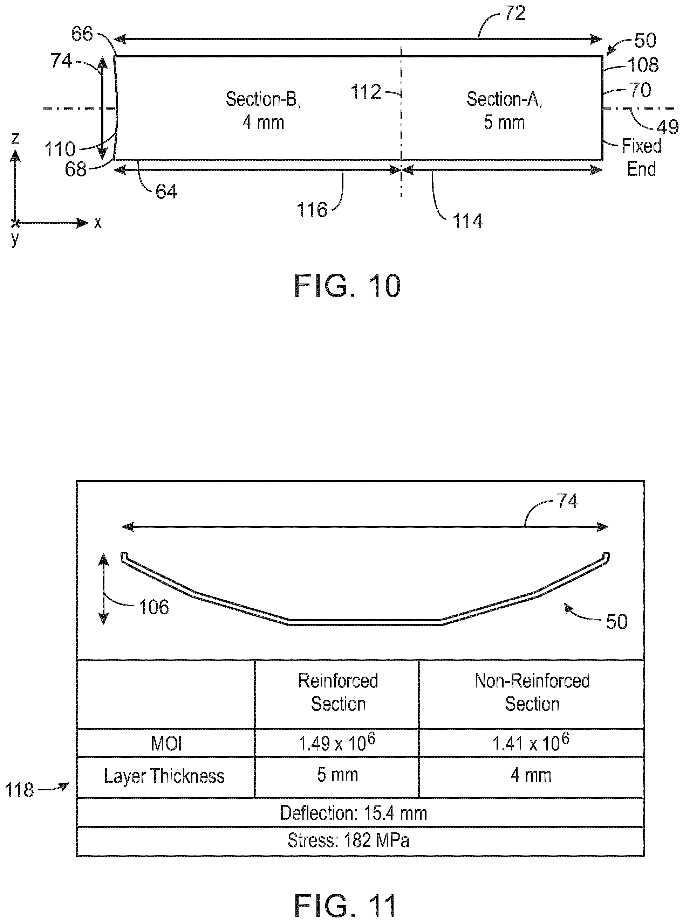

The cradle 50 includes a top surface 100 and a bottom 102. The patient is disposed on the top surface 100 and the bottom surface 102 faces the fixed structure (see FIG. 4). Along the cross-section of the cradle 50, the cradle 50 and each section 90 has a thickness 104 between the top surface 100 and the bottom surface 102. As discussed in greater detail below, the thickness 104 varies along the longitudinal length 72 (see FIG. 10). The variability in thickness 104 along the longitudinal length 72 in conjunction with the variability in the profile or cross-section of cradle 50 distributes the deflection and the stresses across the cradle 50. The thickness 104 may also vary along the width 74.

FIG. 8 is an end view of the cradle 50 of the patient table depicted in FIG. 2 (e.g., having a first width). FIG. 9 is an end view of the cradle 50 of the patient table depicted in FIG. 2 (e.g., having a second width). The cradle 50 is as described in FIGS. 4 and 5. In FIG. 8, the width 74 of the cradle 50 is 54 cm (540 millimeters (mm)). In FIG. 9, the width 74 of the cradle 50 is 52 cm (520 mm). In certain embodiments, the width 74 may vary between 420 and 560 mm. As depicted in both FIGS. 8 and 9 the cradle 50 includes a depth 106 in the y-direction. In certain embodiments, the depth 106 may vary. The depth 106 and the width 74 of the cradle 50 make the cradle 50 more comfortable to the patient. The cradles 50 in FIG. 8 are of a light weight design that both weighs less and has deflection values less than form core cradles.

FIG. 10 is a top view of the cradle 50 of the patient table depicted in FIG. 2 (e.g., having respective dimensions). The cradle 50 has the profile of the cradle in FIG. 9. The cradle 50 includes a first side 64 and a second side 66 both extending between a first end 68 (first longitudinal end) and a second end 70 (second longitudinal end) in the x-direction. The first end 68 is configured to face the gantry of the imaging system and configured to be moved in and out of the bore of the gantry. The second end 70 (i.e., the fixed end) remains outside the bore of the gantry and remains disposed above the fixed structure (e.g., fixed structure 78 in FIG. 2) when the first end 68 is disposed with the bore of the gantry. The cradle 50, the first side 64, and the second side 66 have the length 72 (e.g., longitudinal length) in the x-direction. In certain embodiments, the length 72 may vary. The cradle 50, the first end 68, the second end 70 have the width 74 in the z-direction. In certain embodiments, the width 74 is at least 42 cm. In certain embodiments, the width 74 may range between 42 cm and 56 cm (or greater).

The cradle 50 varies in thickness (e.g., thickness 104 in FIG. 4) along the longitudinal axis 49 (and the longitudinal length 72). The cradle 50 includes a first section 108 (Section A) and a second section 110 (Section B). Dashed line 112 indicates the division between the first section 108 and the second section 110. The first section 108 is associated with the second end 70. The second section 110 is associated with the first end 68. The first section 108 has a length 114. The second section 110 has a length 116. As depicted, the length 116 of the second section 110 is greater than the length 114 of the first section 108. The lengths 114, 116 of the sections 108, 110 may vary. The length 116 of the second section 110 is greater than the length 114 of the first section 108. As depicted, the ratio of the length 116 to the length 114 is slightly less than 2 to 1. In certain embodiments, the ratio of the length 116 to the length 114 may vary. The thickness (e.g., thickness 104 in FIG. 4) of the first section 108 is greater than the thickness (e.g., thickness 104 in FIG. 4) of the second section 110. As depicted, the thickness of the first section 108 is 5 mm. As depicted, the thickness of the second section 110 is 4 mm. The first section 108 is considered a reinforced section due to the greater thickness. The second section 110 is considered a non-reinforced section. The respective thicknesses of the first section 108 and the second section 110 may vary. In certain embodiments, the first section 108 and the second section 100 may have the same thickness. In certain embodiments, the thicknesses of the first section 108 and the second section 110 besides varying along the longitudinal length 72 may also vary along the width 74.

The variability in thickness 104 along the longitudinal length 72 in conjunction with the variability in the profile or cross-section of cradle 50 distributes the deflection and the stresses across the cradle 50. FIG. 11 is an end view of the cradle 50 in FIG. 10 and table 118 with associated values. As indicated in the table 118, the reinforced section (e.g., first section 108 in FIG. 10) has a thickness of 5mm. The non-reinforced section (e.g., second section 110 in FIG. 10) has a thickness of 4 mm. The reinforced section has a moment of inertia (MOI) of 1.49 x 10⁶. The non-reinforced section has a moment of inertia of 1.41 x 10⁶. Overall, the cradle 50 has a deflection of 15.4 mm. Also, the cradle 50 has a stress of 182 megapascal (MPa). The deflection and stress experienced by the cradle 50 is well within acceptable limits.

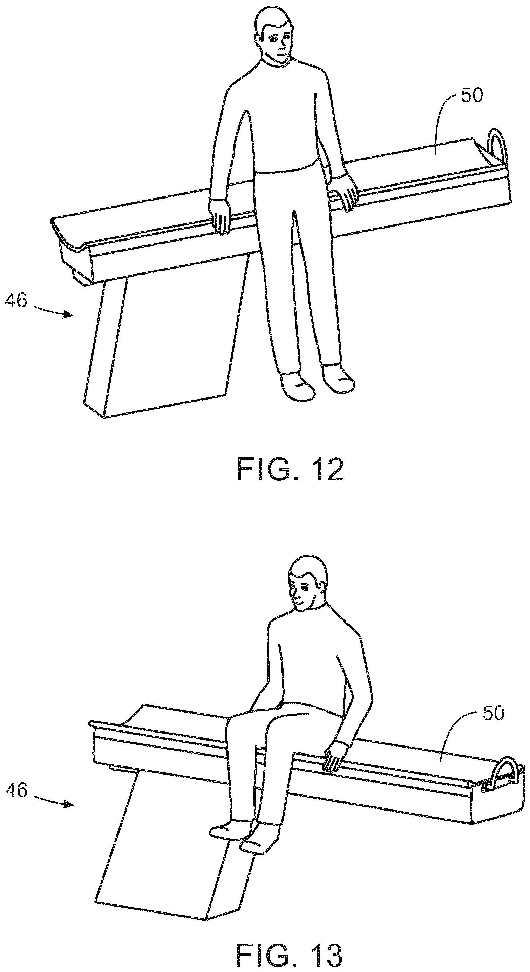

As noted above, the cradle 50 is configured to support a single subject (patient) point load to enable the subject to load and to unload onto the cradle 50 solely utilizing the cradle 50. In particular, as depicted in FIGS. 12 and 13, the cradle 50 experiences a load when the subject 22 (patient) loads on the cradle 50 with a concentrated load via one or more their hands putting their entire loading weight on 1 or 2 points on a small area.

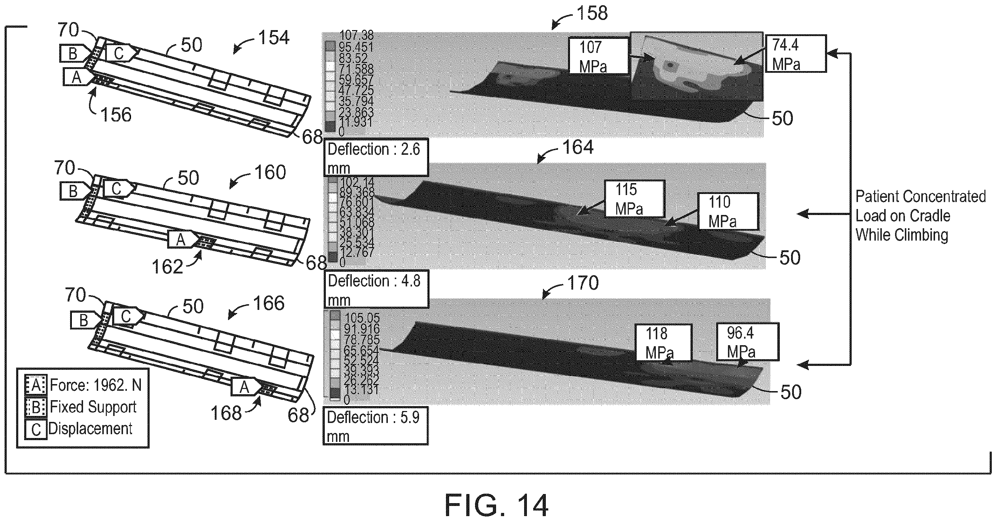

FIG. 14 depicts analysis of a load case (e.g., patient climb load case) for the cradle 50 in FIG. 10. The boundary condition for the patient climb load case is that the cradle 50 is considered to be in an out of gantry position. A concentrated load of 200 kg is applied over 150 * 150 mm². Also, each side of the cradle 50 is supported by 4 rollers. Image 154 indicates a first area 156 (closer to end 70) where the concentrated load is applied along the side of the cradle. 50 Image 158 depicts a corresponding stress plot of the cradle 50 in response to the concentrated load being applied in the first area 156. The highest stresses applied to the cradle 50 were 74.4 MPa and 107 MPa. The deflection experienced by the cradle 50 is 2.6 mm.

Image 160 indicates a second area 162 (more central) where the concentrated load is applied along the side of the cradle 50. Image 164 depicts a corresponding stress plot of the cradle 50 in response to the concentrated load being applied in the second area 162. The highest stresses applied to the cradle 50 were 110 MPa and 115 MPa. The deflection experienced by the cradle 50 is 4.8 mm.

Image 166 indicates a third area 168 (closer to the end 68) where the concentrated load is applied along the side of the cradle 50. Image 170 depicts a corresponding stress plot of the cradle 50 in response to the concentrated load being applied in the third area 168. The highest stresses applied to the cradle 50 were 96.4 MPa and 118 MPa. The deflection experienced by the cradle 50 is 5.9 mm.

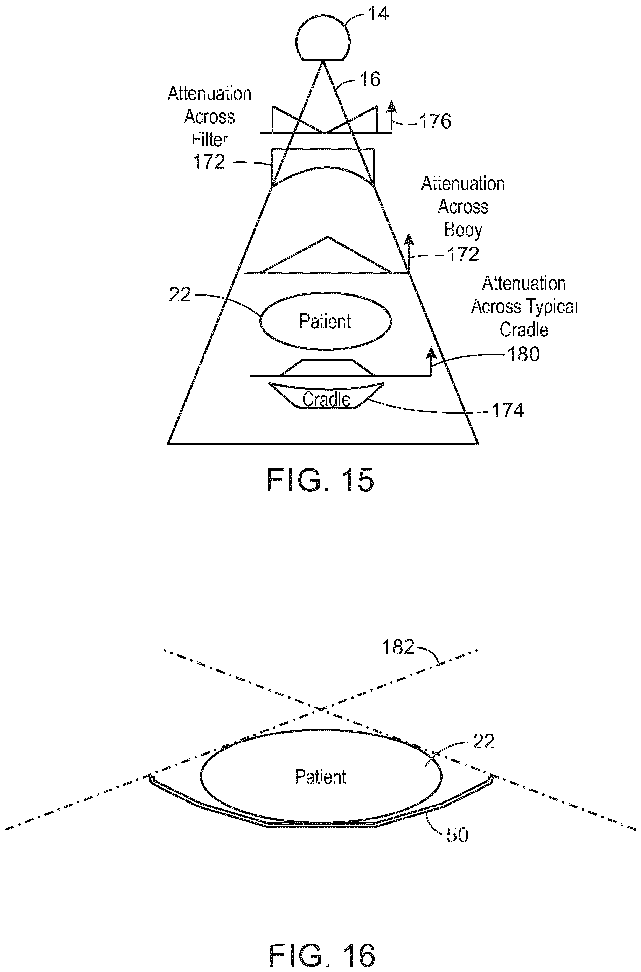

FIG. 15 depicts the attenuation at various points during imaging (e.g., with a CT imaging system 10) of the patient 22 utilizing a typical cradle. FIG. 15 depicts the X-ray source 14 and the beam of X-rays 16 emitted from the X-ray source. Depicted within the path of the beam of X-rays 16 are a filter 172 (e.g., bowtie filter), the body of the patient 22, and a typical cradle 174. The patient 22 is disposed between the filter 172 and the cradle 174. The cradle 174 is disposed underneath the subject 22. The filter 172 is disposed between the X-ray source 14 and the body of the patient 22. Graph 176 depicts the attenuation across the filter 172. Graph 178 depicts the attenuation across the body of the patient 22. Graph 180 depicts attenuation across the cradle 174. The typical cradle 174 is thicker (e.g., due to the foam core) than the cradle disclosed in the present disclosure. As result, the typical cradle has a higher attenuation which results in a higher X-ray dose for the patient 22.

FIG. 16 depicts the cradle 50 of the patient table depicted in FIG. 2 with respect to path of X-rays passing through the patient 22 during a scan of the patient 22. As noted above, the cradle 50 is made of carbon fiber reinforced plastic. In certain embodiments, the cradle 50 lacks a foam core. The thinner and slimmer configuration of the cradle 50 (compared to the typical cradle such as cradle 174 in FIG. 15) ensures that the amount of composite material is minimal that is disposed in the path traced by the X-rays 182 passing through the patient 22 as depicted in FIG. 16.

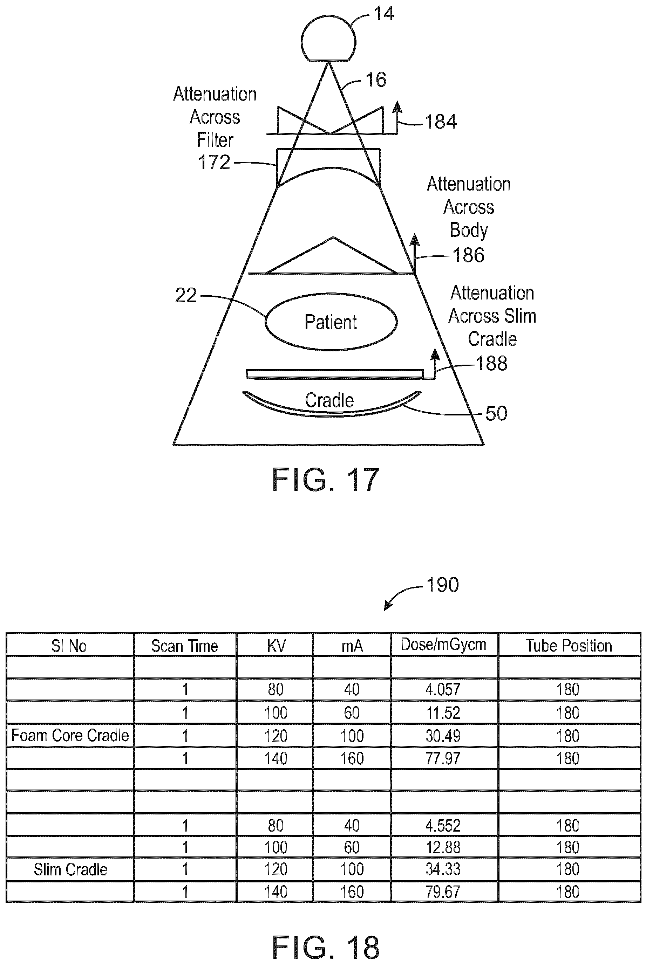

FIG. 17 depicts the attenuation at various points during imaging (e.g., with a CT imaging system 10) of the patient 22 utilizing the cradle 50 of the patient table depicted in FIG. 2. FIG. 17 depicts the X-ray source 14 and the beam of X-rays 16 emitted from the X-ray source. Depicted within the path of the beam of X-rays 16 are the filter 172 (e.g., bowtie filter), the body of the patient 22, and the cradle 50. The patient 22 is disposed between the filter 172 and the cradle 50. The cradle 50 is disposed underneath the subject 22. The filter 172 is disposed between the X-ray source 14 and the body of the patient 22. Graph 184 depicts the attenuation across the filter 172. Graph 186 depicts the attenuation across the body of the patient 22. Graph 188 depicts attenuation across the cradle 50. As noted above, the cradle 50 is both thinner and slimmer than the typical cradle (e.g., cradle 174 in FIG. 15). In addition, the cradle 50 is wider than the typical cradle. Further, in certain embodiments, the cradle 50 lacks a foam core. As noted in FIG. 16, the disclosed configuration of the cradle 50 (compared to the typical cradle such as cradle 174 in FIG. 15) ensures that the amount of composite material is minimal that is disposed in the path traced by the X-rays passing through the patient 22. The attenuation in across the cradle 50 (as depicted in the graph 188) is significantly less comparted to the attenuation across the typical cradle (as depicted in the graph 180 in FIG. 15). The lower attenuation across the cradle 50 results in a lower X-ray dose for the patient 22 (compared to when utilizing a typical cradle).

FIG. 18 depicts a table 190 of results for analyzing X-ray attenuation for both a typical cradle and the cradle 50 of the patient table depicted in FIG. 2. Both a typical cradle (e.g., referred to as a foam core cradle in the table 190) having a foam core (e.g., cradle 177 in FIG. 15) and the cradle 50 (e.g., referred to as a slim cradle in the table 190) lacking a foam core. A portion of the cradle 50 was three-dimensionally printed and utilized in comparisons tests with the typical cradle. Both the typical cradle and the cradle 50 were subject to CT scans with a CT imaging system under different kilovolts (kV) and milliampere (mA) parameters for the X-ray source with the X-ray source located underneath the respective cradles. A dosimeter was placed on both the typical cradle and the cradle 50 to measure the dose. As depicted in the table 190, a higher dose was measured with the cradle 50 compared to the typical cradle, thus, showing that the X-ray dose for the patient with the cradle 50 will be lower than with the typical cradle.

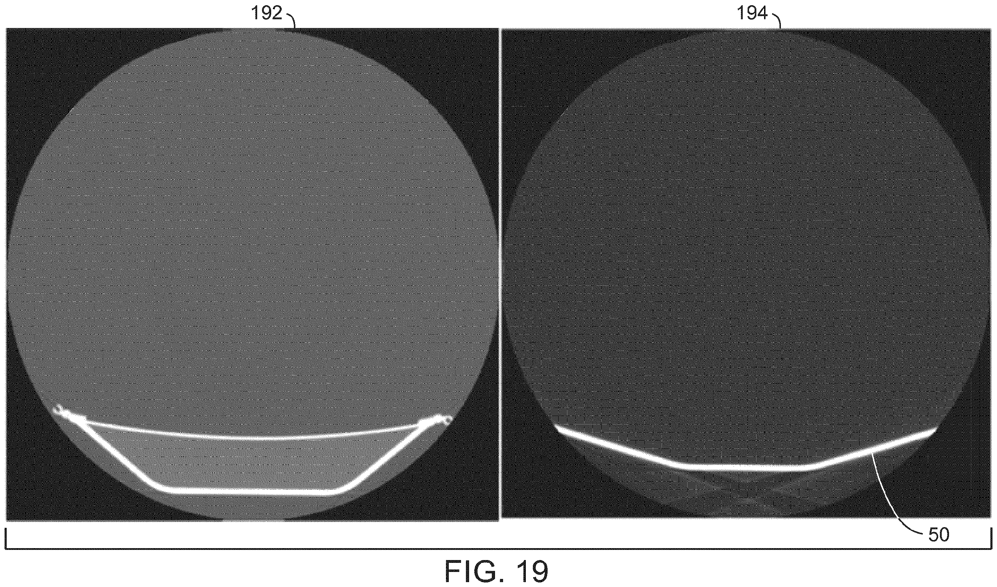

In addition, the image quality with the typical cradle having a foam core and the cradle 50 lacking a foam core was checked. FIG. 19 depicts CT images of both the typical cradle and the cradle 50 of the patient table depicted in FIG. 2. Both the typical cradle and the cradle 50 were subject to a CT scan with a CT imaging system with 80 kV and 350 mA for the X-ray source parameters. Image 192 is a CT image of the typical cradle. Image 194 is of 196 is of the cradle 50. The image quality of the cradle 50 is of similar quality to the typical cradle.

As noted above, the wide angle, pinchless patient positioning and patient supporting may also be utilized with a patient table for other types of medical imaging systems (e.g., magnetic resonance imaging (MRI), positron emission tomography (PET) imaging system, single-photon emission computed tomography (SPECT) imaging system, nuclear medicine imaging system, X-ray imaging system, etc.).

Technical effects of the disclosed subject matter include providing a patient table having a cradle and lacking side support structures to provide a pinchless configuration where no portion of a subject will be pinched between the cradle and the fixed structure during movement of the cradle relative to the fixed structure. The cradle itself serves as the support structure for patient positioning on the cradle without the need of the side support structures. The disclosed configuration of the table eliminates any need for additional external support or structure to hold the cradle and position the cradle. The elimination of the side support structures and the wider cradle keeps the patient from experiencing kinesiophobia and claustrophobia. Technical effects of the disclosed subject matter further include providing a patient table that does not provide a cold shock to the patient. Thus, the disclosed subject matter provides a better patient experience. Technical effects of the disclosed subject matter also include providing a patient able with improved cleanability and disinfection. This will reduce associated repair issues and, thus, less downtime for the medical imaging system. Technical effects of the disclosed subject matter even further include providing a wider cradle that a flat surface for engagement with a radiation therapy table top. Technical effects of the disclosed subject matter still further include providing a thin, strong, and cost efficient part (i.e., the cradle) made of carbon fiber reinforced plastic. A simple carbon fiber reinforced plastic lay-up process for manufacturing the cradle improves production.. Technical effects of the disclosed subject matter event further include subjecting a patient to a lower X-ray dose due to the cradle having a lower X-ray attenuation. This improves the life of the X-ray tube and improves the overall system throughput.

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the subject matter, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A patient table (46) for a medical imaging system, comprising:
a base (52);
a cradle (50) configured to support a subject to be imaged and to move bi-directionally relative to the base (52); and
a fixed structure (78) coupled to both the base (52) and the cradle (50), wherein the cradle (50) is configured to move relative to the fixed structure (78) without a portion of the subject being pinched between the cradle (50) and the fixed structure (78).

2. The patient table (46) of claim 1, wherein the cradle (50) has a first width in a first direction perpendicular to a longitudinal axis of the cradle (50), and the first width is greater than a second width of the fixed structure (78) in the first direction.

3. The patient table (46) of claim 2, wherein the cradle (50) is located above the fixed structure (78) along the first width.

4. The patient table of claim 2, wherein the first width is at least 42 centimeters.

5. The patient table (46) of claim 2, wherein a cross-section of the cradle (50) along the first width comprises a plurality of sections with at least two sections sloped relative to a central section of the plurality of sections.

6. The patient table (46) of claim 2, wherein the cradle (50) varies in thickness along the longitudinal axis in both in the first direction and a second direction perpendicular to the first direction.

7. The patient table (46) of claim 6, wherein the cradle (50) comprises a first section and a second section along the longitudinal axis, the first section is configured to be extended beyond the fixed structure (78) while the second section is configured to remain located above the fixed structure (78) when the first section is extended beyond the fixed structure, the first section has a first thickness in the second direction along the longitudinal axis and the second section has a second thickness in the second direction along the longitudinal axis, and the second thickness is greater than the first thickness.

8. The patient table (46) of claim 1, wherein the cradle (50) is configured to support a single subject point load to enable the subject both to load and to unload onto cradle (50) solely utilizing the cradle (50).

9. The patient table (46) of claim 1, wherein the cradle (50) is configured to provide a flat surface for engagement with a radiation therapy table top.

10. The patient table (46) of claim 1, wherein the cradle (50) lacks a foam core, and the cradle (50) has a lower X-ray attenuation than another cradle having the foam core.

11. The patient table (46) of claim 10, wherein the cradle (50) comprises a foam core.

12. A computed tomography (CT) imaging system, comprising:
a gantry having a bore and coupled to imaging components configured to acquire imaging data of a subject; and
a patient table (46) comprising:
a base (52);
a cradle (50) configured to support a subject to be imaged and to move bi-directionally relative to the base (52); and
a fixed structure (78) coupled to both the base (52) and the cradle (50), wherein the cradle (50) has a first width in a first direction perpendicular to a longitudinal axis of the cradle (50), and the first width is greater than a second width of the fixed structure (78) in the first direction.

13. The CT imaging system of claim 12, wherein the cradle (50) is located above the fixed structure (78) along the first width.

14. The CT imaging system of claim 12, wherein the first width is at least 42 centimeters.

15. The CT imaging system of claim 12, wherein a cross-section of the cradle (50) along the first width comprises a plurality of sections with at least two sections sloped relative to a central section of the plurality of sections.
